# EUROPEAN PATENT SPECIFICATION

(11) **EP 1 693 056 B1**
(45) Date of publication and mention of the grant of the patent: **24.12.2008**
(21) Application number: 05253177.9
(22) Date of filing: 24.05.2005
(51) Int. Cl.: A61K 9/48

(54) **Cellulose hard capsule shell with enhanced mechanical film strength containing iota-carrageenan and agar**
Cellulose Hartkapselhülle mit verbesserter mechanischer Filmfestigkeit enthaltend Iota-carrageenaan und Agar
Enveloppe de capsule dure avec une résistance mécanique améliorée contenant du iota-carrageenine et de l'agar

(30) Priority: 21.02.2005 KR 2005014135
(43) Date of publication of application: 23.08.2006
(73) Proprietor: Suheung Capsule Co Ltd., Bucheon, Kyonggi-do (KR)
(72) Inventor: Yang, Joo Hwan, Bucheon Kyonggi-do (KR)
(74) Representative: Simcox, Michael Thomas

(56) References cited:
- WO-A-03/011257
- US-A- 5 264 223
- US-B1- 6 410 050
- US-B2- 6 517 865
- BCHEALTH FILES: "Food sources of calcium and vitamin D"[Online] November 2005 (2005-11), pages 1-2, XP002360385 Retrieved from the Internet: URL:http://www.bchealthguide.org/healthfil es/hfile68e.pdf> [retrieved on 2005-12-21]

## Description

### BACKGROUND OF THE INVENTION

The present invention relates to a cellulose hard capsule with enhanced mechanical film strength, prepared by adding a specific polysaccharide gelling agent to a cellulose base material and its preparation method. More particularly, this invention relates to a cellulose hard capsule with enhanced mechanical film strength prepared by the steps comprising i) adding sucrose fatty acid ester, potassium pyrophosphate and glacial acetic acid to a cellulose base material solution; ii) adding a mixed solution of iota-carageenan and agar as gelling agent to the mixture obtained; and iii) allowing the product obtained to stand, adjusting its viscosity and forming a capsule from it.

The traditional material for forming the capsule is gelatin, because it has the correct and quite ideal properties. Nevertheless, gelatin has some disadvantages which make it necessary to have other capsule materials available. A major unfavorable aspect is the animal origin of gelatin which makes it unsuitable for use by vegetarians. Other disadvantages of gelatin are the inconveniences of its relatively high water content (10-16%) and its loss of elasticity with decreasing water content. Furthermore gelatin capsules are sensitive to heat and humidity which affects the usefulness of the product.

As a gelatin substitute, cellulose hard capsules have been disclosed. Cellulose hard capsules are used to encapsulate hygroscopic material because the water content of cellulose hard capsules are less than 7% which is lower than that of gelatin capsules. Further, cellulose avoids the problem of protein denaturation that may occur with gelatin because cellulose originates from plants. Of course, it may also be used by vegetarians.

However, there are some handicaps with cellulose hard capsules, gelation of a cellulose solution is less efficient than for gelatin which results in a lower film strength and lower stability for cellulose hard capsules. To improve the gelation of a cellulose solution, methods for adding various polysaccharides to a cellulose solution have been disclosed.

In U.S. Pat. No. 5,431,917, cellulose hard capsules for pharmaceutical drugs were disclosed. In the examples of this disclosure, kappa-carrageenan carrageenan was disclosed as a preferred gelatinizing agent and potassium chloride was disclosed as a preferred auxiliary for gelation.

On the other hand, in U.S. Pat. No. 6,517,865 B2, a capsule film composition comprising i) 90-99.98 wt% of at least one cellulose ether, ii) 0.01-5 wt% of gellan gum, and iii) 0.01-8 wt% of sequestering agent selected from ethylene diamine tetraacetic acid (EDTA), sodium citrate, citric acid or a combination thereof was disclosed.

Further, U.S. Pat. No. 6,41,0,050 B1, disclosed a cellulose hard capsule prepared by a process comprising i) preparing a mixed solution of pectin and glycerin; ii) adding said mixed solution to a solubilized cellulose aqueous solution; iii) adding glacial acetic acid, calcium gluconate and a sucrose fatty acid ester to said mixture; and iv) allowing the product obtained to stand, adjusting its viscosity and forming a capsule.

U.S. Pat. No. 5,264,223 discloses a hard capsule for pharmaceutical drugs comprising a water-soluble cellulose derivative as a capsule base, a gelatinizing agent and an auxiliary for gelation.

WO 03011257 discloses a cellulose ether soluble container composition comprising high molecular weight cellulose ether blended with low molecular weight cellulose ether, the blend mixed along with one or more among gelling agent, sequestering agent and/or co-gelling agent.

However, all cellulose hard capsules prepared by the above disclosed methods have the drawbacks of a lower mechanical film strength and a lower oxygen transparency compared to those of a conventional gelatin hard capsule.

The reason why cellulose hard capsules have a lower mechanical film strength is because of the physical properties of cellulose. Thus, the following problems occur at the time of preparing a cellulose hard capsule and filling the drug or food in to this capsule.

First, a weak joint is formed when the film of a cap and the film of a body, formed separately, are joined at the joiner's block because of the low mechanical film strength of the cellulose film.

Second, mashed waste occurs when capsules are transferred among drums in a capsule inspection machine, a capsule printing machine and/or a capsule filling machine because of the low mechanical film strength of the cellulose film.

Third, mashed waste occurs when capsules are pushed by a rubber roll in order to transcribe the ink from the rubber roll to a surface of the capsule during the spin printing process because of the low mechanical film strength of the cellulose film.

Fourth, a telescoping problem occurs when a cap and body of a capsule are joined after filling the food or drug in to the capsule because of low mechanical film strength of cellulose film.

Therefore, enhancement of the film strength is required for the commercial use of cellulose hard capsules. The present invention accomplished the enhancement of film strength of cellulose hard capsules by adding a specific polysaccharide gelling agent to a cellulose base material. Further, the present invention provides cellulose hard capsules with enhanced film strength, which also reduces the problems of weak joints, mashed waste and/or telescoping problems that can occur during the preparation of cellulose hard capsules.

### SUMMARY OF THE INVENTION

The object of the present invention is to provide carrageenan a process for preparing a cellulose capsule with enhanced mechanical film strength, comprising the steps of : i) preparing a 100 wt parts of an aqueous solution containing 18-21 wt parts of solubilized cellulose; ii) adding 0.1-0.5 wt parts of sucrose fatty acid ester, 0.05-0.3 wt parts of potassium pyrophosphate and 0.01-0.2 wt parts of glacial acetic acid to the 100 wt parts aqueous solution of solubilized cellulose; iii) adding a mixed solution of 0.1-1.0 wt parts of iota-carrageenan carrageenan and 0.02-0.5 wt parts of agar to the resulting admixture; and iii) allowing the product obtained to stand, adjusting its viscosity and forming a capsule from it.

The weight ratio of iota-carrageenan and agar is preferably 3.5-4.5 : 1 and said cellulose is preferably hydroxypropylmethylcellulose (HPMC).

Said glacial acetic acid is added to neutralize the pH of an alkaline solution of solubilized cellulose.

In another aspect, the present invention provides a cellulose hard capsule comprising 100 wt parts of cellulose, 0.5-2.5 wt parts of sucrose fatty acid ester, 0.2-1.5 wt parts of potassium pyrophosphate, 0.05-0.3 wt parts of glacial acetic acid, 1.0-5.0 wt parts of iota-carrageenan and 0.1-2.5 wt parts of agar.

In another aspect, the present invention provides a cellulose hard capsule prepared by above preparation method comprising 100 wt parts of cellulose, 0.5-2.5 wt part of sucrose fatty acid ester, 0.2-1.5 wt parts of potassium pyrophosphate, 0.05-0.3 wt parts of glacial acetic acid, 1.0-5.0 wt parts of iota-carrageenan and 0.1-2.5 wt parts of agar.

### BRIEF DESCRIPTION OF THE DRAWINGS

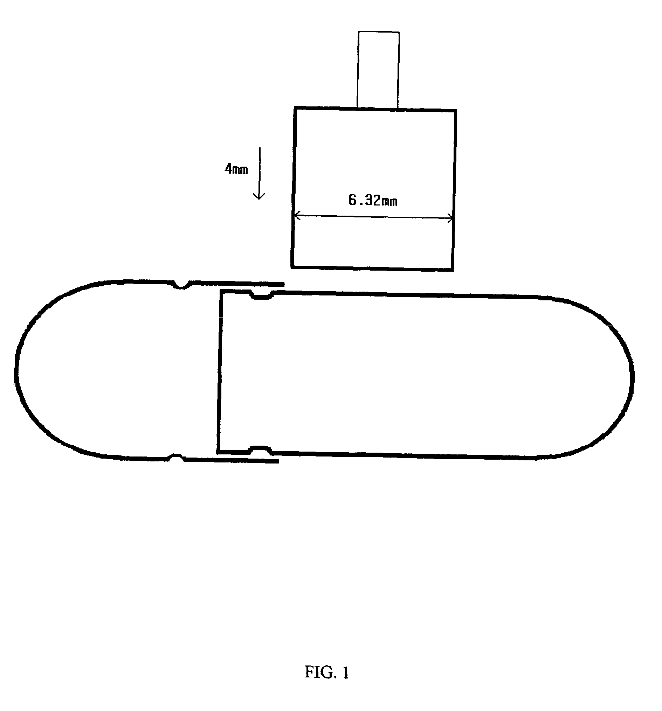

FIG. 1 shows a schematic view indicating a process and apparatus for detecting the mechanical film strength of a cellulose hard capsule of the present invention. The film strength is measured by dropping and pressing with the hammer at 4 mm depth from surface of capsule.

### DETAILED DESCRIPTION OF THE INVENTION

For preparing conventional cellulose hard capsules, a physical gelling method or a chemical gelling method can be employed after dipping a molding pin in a solubilized cellulose solution.

The physical gelling method uses a heated (to more than 67°C) molding pin for gelling the solubilized cellulose solution. On the other hand, the chemical gelling method requires the addition of a polysaccharide gelling agent to the solubilized cellulose solution.

Even though the cellulose film strength for conventional capsules prepared by the physical gelling method is higher than that of the chemical gelling method, the cellulose film strength of capsules prepared by the physical gelling method is still lower than that of a gelatin film. Table 1 shows the data of the cellulose film strength, according to gelling method used, in comparison to the gelatin film strength.

**Table 1**

| Items | | | Film strength (g) | | |
|---|---|---|---|---|---|
| | | | Max. | Mean | Min. |
| Gelatin hard capsule | | | 561 | 468 | 417 |
| Cellulose hard capsule | Physical gelling method | | 448 | 356 | 290 |
| | Chemical gelling method | Pectin | 292 | 234 | 200 |
| | | Gellan gum | 343 | 300 | 276 |
| | | Carrageenan | 382 | 338 | 280 |

The mechanical film strength is measured by Texture Analyser (Model TA 1000). The dropping speed of the measuring hammer is 0.5 mm/sec and the depth of measurement of the mechanical film strength is 4 mm from the surface of the capsule. The testing capsule is laid on Texture Analyser and the film strength is measured at 4 mm pressing depth by dropping the hammer.

As shown in Table 1, the mechanical film strength of a conventional cellulose hard capsule prepared by the physical gelling method is higher than that prepared by chemical gelling method. Nevertheless, most capsule manufacturers prefer to use the chemical gelling method, because the chemical gelling method does not require the alteration of the molding pin or other capsule manufacturing machines, whereas the physical gelling method requires the additional cost for alteration of the molding pin and of other capsule manufacturing machines.

However, cellulose hard capsules prepared by the chemical gelling method have some of the above mentioned handicaps because of their low mechanical film strength compared to that of gelatin hard capsules.

Carrageenan can be classified into 3 types; kappa-carrageenan, iota-carrageenan and lambda-carrageenan. Of these, kappa-carrageenan and iota-carrageenan can form a double helix structure connecting 2 molecular chains in a 3-dimension structure, which results in the formation of a gel suitable to be used for manufacturing capsules. However, lambda-carrageenan does not form a double helix structure, and cannot be used for manufacturing capsules.

Kappa-carrageenan in particular has been used as a gelatinizing agent, because a strong gel is formed in combination with a potassium ion such as potassium chloride. However, iota-carrageenan can be a better gelatinizing agent when it is mixed with a compatible auxiliary for gelation, because the double helix structure of iota-carrageenan is more compatible for forming a gel.

To investigate the compatibility of iota-carrageenan for forming a gel, various mixed solutions of iota-carrageenan and other polysaccharides were prepared. These mixed solutions were used as a gelatinizing agent in the preparation of sample cellulose capsules which were tested for enhancement of the mechanical film strength.

Other polysaccharide employed for this test can be one or more selected from agar gum, guar gum, locust bean gum, gellan gum, xanthan gum, pectin, starch, pulluran and agar. A mixed solution of iota-carrageenan and agar was the best combination as a gelatinizing agent, providing enhancement of the mechanical film strength of cellulose hard capsules.

The test also indicated that the use of a mixed solution of iota-carrageenan and agar gave better mechanical film strength results for cellulose hard capsules compared to the use of kappa-carrageenan alone or in combination.

The selection of an auxiliary for gelation of iota-carrageenan was also tested among potassium phosphate monobasic, potassium phosphate dibasic, potassium gluconate, potassium citrate, potassium carbonate and potassium pyrophosphate. These tests indicated that potassium pyrophosphate is the best auxiliary for gelation of iota-carrageenan.

Further, glacial acetic acid is used in the formation of gel. The glacial acetic acid adjusts the pH of the cellulose aqueous solution and avoids it becoming alkaline by addition of potassium pyrophosphate. Adding glacial acetic acid adjusts the pH of the cellulose aqueous solution to be about pH 6.

In addition, use of a sucrose fatty acid ester as an emulsifier gave excellent film distribution as well as film strength results.

The present invention will be described in more detail with reference to the following examples. However, it should be understood that the examples are intended to illustrate but not in any manner to limit the scope of the present invention.

### EXAMPLES

### (Preparation Examples 1-3) Preparation of a cellulose hard capsule

Hydroxypropylmethylcellulose (HPMC) is added to purified (de-ionized and distilled) water (at about 80°C). Then, the mixture is stirred and dispersed. Sucrose fatty acid ester, potassium pyrophosphate and glacial acetic acid are also added to the solubilized cellulose solution. Then, a mixed solution of iota-carrageenan and agar is added to the resulting admixture and stirred at 60 rpm for 2 hours. After stirring, the mixed cellulose solution (about 80°C) is cooled to about 45°C. Then, the mixed cellulose solution is heated again until 58°C for molding. Finally, a cellulose hard capsule is formed using a capsule manufacturing machine.

According to above preparation method, cellulose hard capsules have been prepared with the following composition contents as shown in Table 2. The content and ratio of iota-carrageenan and agar have been changed in each sample, relative to a constant content of cellulose base material. On the other hand, the content of sucrose fatty acid ester, potassium pyrophosphate and glacial acetic acid are constant in all of these preparation examples.

**Table 2.**

| Items | Pre. Ex. 1 | Pre. Ex. 2 | Pre. Ex. 3 |
|---|---|---|---|
| HPMC | 94.751 | 94.751 | 94.751 |
| Sucrose fatty acid ester | 0.9475 | 0.9475 | 0.9475 |
| Potassium pyrophosphate | 0.4737 | 0.4737 | 0.4737 |
| Glacial acetic acid | 0.1421 | 0.1421 | 0.1421 |
| Iota-carrageenan | 3.1321 | 3.3163 | 3.5006 |
| Agar | 0.5528 | 0.3686 | 0.1843 |

### (Example 1) Measurement of mechanical film strength of the cellulose hard capsule

The mechanical film strength of a cellulose hard capsule prepared in Preparation Examples 1-3 and of a control have been measured using #0 size capsule. The cellulose hard capsule used as a control has been prepared according to the method disclosed in Example 1 of U.S. Pat. No. 5,431,917. The results of the measurement of mechanical film strength are shown in Table 3.

**Table 3. Comparison of mechanical film strength (No. of samples measured n = 100)**

| Items | | Pre. Ex. 1 | Pre. Ex. 2 | Pre. Ex. 3 | Control | Gelatin hard capsule |
|---|---|---|---|---|---|---|
| Mechanical film strength (g) | Max. | 468 | 452 | 406 | 396 | 561 |
| | Mean | 413 | 405 | 350 | 344 | 468 |
| | Min. | 380 | 364 | 304 | 297 | 417 |

| | | | | | | |
|---|---|---|---|---|---|---|
| * The method for measuring mechanical film strength is the same as the method described in relation to Table 1. | | | | | | |

As shown in Table 3, capsules prepared by Preparation Examples 1 and 2 give a better mechanical film strength than conventional capsules prepared by a physical gelling method (see results in Table 1). This shows that the mechanical film strength increases with an increase in amount of the agar component in the mixture of iota-carrageenan and agar. Further, the capsules prepared in Preparation Examples 1-3 have a better mechanical film strength than that of the control. This shows that iota-carageenan and agar is a better gelatinizing agent than kappa-carrageenan alone.

### (Example 2) Measurement of film transparency of cellulose hard capsules

The film transparency of a cellulose hard capsule prepared by Preparation Examples 1-3 and a control has been measured using a UV-visible ray spectrophotometer at 570 nm. The cellulose hard capsule used as a control has been prepared according to the method disclosed in U.S. Pat. No. 6,410,050 B1. Test samples have been prepared with a length of 1 cm and a width of 1 cm from the body part of the cellulose capsule. The results of the film transparency measurements are shown in Table 4.

**Table 4. Comparison of film transparency**

| Item | Pre. Ex. 1 | Pre. Ex. 2 | Pre. Ex. 3 | Control |
|---|---|---|---|---|
| Transparency (%) | 42% | 50% | 51% | 48% |

As shown in Table 4, the film transparency of capsules prepared by Preparation Examples 2-3 is better than that of the control. However, the film strength of capsules prepared by Preparation Example 3 is not enough to be commercially used. Therefore, a cellulose hard capsule prepared by Preparation Example 2 gives the better overall results when considering both film strength and film transparency.

### (Example 3) Preparation of cellulose hard capsules of the present invention

19.5 Kg of hydroxypropylmethylcellulose (HPMC) (concentration % w/v : 19.5%) is added to 79.915 L of purified (de-ionized and distilled) water (about 80°C). Then, the mixture is stirred and dispersed. 195 g of sucrose fatty acid ester (concentration % w/v : 0.195%), 97.5 g of potassium pyrophosphate (concentration % w/v : 0.0975%) and 29.25 g of glacial acetic acid (concentration % w/v : 0.0292%) are also added to solubilized cellulose solution. Then, a mixed solution of 682.5 g of iota-carrageenan (concentration % w/v : 0.6825%) and 75.855 g of agar (concentration % w/v : 0.0758%) is added to resulting admixture and stirred at 60 rpm for 2 hours. After stirring, the mixed cellulose solution (about 80°C) is cooled to about 45°C. Then, the mixed cellulose solution is heated again until 58°C for molding. Finally, a cellulose hard capsule is formed from capsule manufacturing machine.

### (Example 4) Filling procedure for cellulose hard capsules of the present invention

According to the method disclosed in Example 3, a #0 size transparency hard capsule has been prepared. As a control, the cellulose hard capsule prepared by the method disclosed in U.S. Pat. No. 6,410,050 B1 has been employed. The results of the filling procedure of cellulose hard capsules and of a control are shown in Table 5.

**Table 5. Comparison of filling procedure**

| Type of filling up machine | | EXC-100F | | | |
|---|---|---|---|---|---|
| Filling up velocity | | 50,000/hr | | 100,000/hr | |
| Pressure | | 20 cm Hg | | 20 cm Hg | |
| Amount | | 500,000 pieces | | 500,000 pieces | |
| Result | Items | Control | Present Invention | Control | Present Invention |
| | Telescoping problem | 15 | 1 | 20 | 3 |
| | Mashed waste problem | 8 | 0 | 10 | 1 |

As shown in Table 5, the cellulose hard capsules prepared in Example 3 give less filling problems by reducing the proportion of capsules that have to be rejected due to telescoping and mashed waste problems in the filling procedure. These results show that the film strength of cellulose hard capsules of the present invention is excellent and that these capsules may be commercially used.

### (Example 5) Printing of cellulose hard capsules of the present invention

According to the method disclosed in Example 3, a #0 size transparency hard capsule has been prepared. As a control, the cellulose hard capsule prepared by the method disclosed in U.S. Pat. No. 6,410,050 B1 has been employed. The results of the printing procedure for cellulose hard capsules and control capsules are shown in Table 6.

**Table 6. Comparison of printing procedure**

| Type of printing machine | EXC-100R (Spin printer) | |
|---|---|---|
| Printing velocity | 100,000/hr | |
| Amount | 500,000 pieces | |
| Telescoping or mashed waste problems | Control | Present Invention |
| | 10 pieces | 1 pieces |

As shown in Table 6, the cellulose hard capsules prepared in Example 3 give better results by reducing the proportion of capsules that have to be rejected due to telescoping and mashed waste problems in the printing procedure. These results show that the film strength of cellulose hard capsules of the present invention is excellent and that the capsules may be commercially used.

### (Example 6) Roundness of cellulose hard capsule of present invention

According to the method disclosed in Example 3, a #0 size transparency hard capsule has been prepared. As a control, the cellulose hard capsule prepared by the method disclosed in U.S. Pat. No. 6,410,050 B1 has been employed. The roundness is measured using a profile projector after dividing the capsule into cap and body. The roundness of cellulose hard capsule and control is shown in Table 7.

**Table 7. Comparison of roundness (No. of samples measured n = 50)**

| Item | Control | | Present Invention | |
|---|---|---|---|---|
| Roundness (%) | Cap | Body | Cap | Body |
| | 98.5% | 98.3% | 99.3% | 99.1% |

As shown in Table 7, the cellulose hard capsules prepared in Example 3 give better results when measuring roundness. This indicates that the film strength of the cellulose hard capsules of the present invention is excellent and that the capsules may be commercially used.

As shown in Examples, the results for filling, printing and roundness of cellulose hard capsules of the present invention are all excellent. This indicates that the film strength of a cellulose hard capsule of the present invention is fully enhanced. In addition, the film strength of cellulose hard capsules of the present invention is better than any of previously known capsule disclosed in U.S. Pat. No. 5,431,917 and U.S. Pat. No. 6,410,050 B1.

## Claims

1. A carrageenan process for prepaying a cellulose capsule with enhanced mechanical film strength comprising the steps of :
i) preparing a 100 wt parts of an aqueous solution containing 18-21 wt parts of solubilized cellulose;
ii) adding 0.1-0.5 wt parts of sucrose fatty acid ester, 0.05-0.3 wt parts of potassium pyrophosphate and 0.01-0.2 wt parts of glacial acetic acid to 100 wt parts of an aqueous solution of solubilized cellulose;
iii) adding a mixed solution of 0.1-1.0 wt parts of iota-carrageenan and 0.02-0.5 wt parts of agar to the resulting admixture; and
iv) allowing the product obtained to stand, adjusting its viscosity and forming a capsule from it.

2. The process for preparing a cellulose capsule with enhanced mechanical film strength according to claim 1, wherein the weight ratio of iota-carrageenan and agar is 3.5-4.5 : 1.

3. The process for preparing a cellulose capsule with enhanced mechanical film strength according to claim 1, wherein said cellulose is hydroxypropylmethylcellulose (HPMC).

4. The process for preparing a cellulose capsule with enhanced mechanical film strength according to claim 1, wherein said glacial acetic acid is added to neutralize the pH of an alkaline solution of solubilized cellulose.

5. A cellulose hard capsule obtainable by the preparation method of claim 1.

6. The cellulose hard capsule according to claim 5, wherein said cellulose hard capsule comprises 100 wt parts of cellulose, 0.5-2.5 wt parts of sucrose fatty acid ester, 0.2-1.5 wt parts of potassium pyrophosphate, 0.05-0.3 wt parts of glacial acetic acid, 1.0-5.0 wt parts of iota-carrageenan and 0.1-2.5 wt parts of agar.

## Patentansprüche

1. Carrageenan-Verfahren zur Herstellung einer Zellulosekapsel mit verbesserter mechanischer Filmfestigkeit, welches die folgenden Stufen umfaßt:
i) Herstellen von 100 Gew.-teilen einer wäßrigen Lösung, die 18-21 Gew.-teile solubilisierte Zellulose enthält,
ii) Zugeben von 0,1-0,5 Gew.-teilen Saccharose-Fettsäureester, 0,05-0,3 Gew.teilen Kaliumpyrophosphat und 0,01-0,2 Gew.-teilen Eisessig zu 100 Gew.-teilen einer wäßrigen Lösung von solubilisierter Zellulose,
iii) Zugeben einer gemischten Lösung von 0,1-1,0 Gew.-teilen lota-Carrageenan und 0,02-0,5 Gew.-teilen Agar zu dem resultierenden Gemisch und
iv) Stehenlassen des erhaltenen Produkts, Einstellen seiner Viskosität und Bilden einer Kapsel daraus.

2. Verfahren zur Herstellung einer Zellulosekapsel mit verbesserter mechanischer Filmfestigkeit nach Anspruch 1, wobei das Gewichtsverhältnis von lota-Carrageenan zu Agar 3,5-4,5 : 1 ist.

3. Verfahren zur Herstellung einer Zellulosekapsel mit verbesserter mechanischer Filmfestigkeit nach Anspruch 1, wobei die Zellulose Hydroxypropylmethylzellulose (HPMC) ist.

4. Verfahren zur Herstellung einer Zellulosekapsel mit verbesserter mechanischer Filmfestigkeit nach Anspruch 1, wobei der Eisessig zugegeben wird, um den pH-Wert einer alkalischen Lösung von solubilisierter Zellulose zu neutralisieren.

5. Zellulosehartkapsel, die durch das Herstellungsverfahren nach Anspruch 1 erhältlich ist.

6. Zellulosehartkapsel nach Anspruch 5, wobei die Zellulosehartkapsel 100 Gew.-teile Zellulose, 0,5-2,5 Gew.-teile Saccharose-Fettsäureester, 0,2-1,5 Gew.-teile Kaliumpyrophosphat, 0,05-0,3 Gew.-teile Eisessig, 1,0-5,0 Gew.-teile lota-Carrageenan und 0,1-2,5 Gew.-teile Agar umfaßt.

## Revendications

1. Procédé utilisant de la carraghénine pour la préparation d'une capsule de cellulose ayant une résistance mécanique accrue de film, comprenant les étapes consistant à :
i) préparer 100 parties en poids d'une solution aqueuse contenant 18 à 21 parties en poids de cellulose solubilisée ;
ii) ajouter 0,1 à 0,5 partie en poids d'un ester d'acide gras de saccharose, 0,05 à 0,3 partie en poids de pyrophosphate de potassium et 0,01 à 0,2 partie en poids d'acide acétique cristallisable à 100 parties en poids d'une solution aqueuse de cellulose solubilisée ;
iii) ajouter une solution constituée d'un mélange de 0,1 à 1,0 partie en poids de iota-carraghénine et 0,02 à 0,5 partie en poids de gélose au mélange résultant ; et
iv) laisser le produit obtenu au repos, ajuster sa viscosité et former une capsule à partir de ce produit.

2. Procédé pour la préparation d'une capsule de cellulose ayant une résistance mécanique accrue de film suivant la revendication 1, dans lequel le rapport pondéral de la iota-carraghénine à la gélose est de 3,3-4,5:1.

3. Procédé pour la préparation d'une capsule de cellulose ayant une résistance mécanique accrue de film suivant la revendication 1, dans lequel ladite cellulose est l'hydroxypropylméthylcellulose (HPMC).

4. Procédé pour la préparation d'une capsule de cellulose ayant une résistance mécanique accrue de film suivant la revendication 1, dans lequel ledit acide acétique cristallisable est ajouté pour neutraliser le pH d'une solution alcaline de cellulose solubilisée.

5. Capsule dure de cellulose pouvant être obtenue par le procédé de préparation de la revendication 1.

6. Capsule dure de cellulose suivant la revendication 5, ladite capsule dure de cellulose comprenant 100 parties en poids de cellulose, 0,5 à 2,5 parties en poids d'un ester d'acide gras de saccharose, 0,2 à 1,5 partie en poids de pyrophosphate de potassium, 0,05 à 0,03 partie en poids d'acide acétique cristallisable, 1,0 à 5,0 parties en poids de iota-carraghénine et 0,1 à 2,5 parties en poids de gélose.
